# EUROPÄISCHE PATENTSCHRIFT

(11) **EP 0 623 680 B1**
(45) Veröffentlichungstag und Bekanntmachung des Hinweises auf die Patenterteilung: **13.01.1999**
(21) Anmeldenummer: 94105668.1
(22) Anmeldetag: 13.04.1994
(51) Int. Cl.: C12P 21/02, C07K 1/02

(54) **Verfahren zur Herstellung von Peptiden und Verwendung**
Process to synthesize peptides and its use
Procédé pour la synthèse des peptides et son utilisation

(30) Priorität: 23.04.1993 DE 4313440; 31.07.1993 DE 4325746
(43) Veröffentlichungstag der Anmeldung: 09.11.1994
(73) Patentinhaber: Degussa Aktiengesellschaft, 60311 Frankfurt (DE)
(72) Erfinder: Jakubke, Hans-Dieter, Prof. Dr., D-04279 Leipzig (DE); Ullmann, Dirk, D-04315 Leipzig (DE); Drauz, Karlheinz, Prof. Dr., D-63579 Freigericht (DE); Bommarius, Andreas, Dr., D-60323 Frankfurt am Main (DE)

(56) Entgegenhaltungen:
- WO-A-91/19811
- "Methods of Enzymatic Analysis", vol. V, "Enzymes III", pages 74-80: "Proteinases", 1984, Bergmayer.

## Beschreibung

Die Erfindung betrifft ein Verfahren zur biokatalytischen Herstellung von Peptiden und die Verwendung von Biokatalysatoren.

Zur Herstellung von Peptiden können verschiedene organisch-chemische Verfahren (vgl. Übersicht: WÜNSCH, E. (1974) Synthese von Peptiden, in HOUBEN-WEYL, Bd. 15, 1/2, Methoden der organischen Chemie, Müller, E. (Hrsg.) Georg Thieme Verlag, Stuttgart) eingesetzt werden. Im Verlauf chemischer Peptidsynthesen werden häufig unerwünschte Nebenreaktionen beobachtet, die die Ausbeute verringern und schwierige und langwierige Reinigungsprozesse erforderlich machen. Ein besonders schwerwiegender Nachteil der herkömmlichen Verfahren ist das ungelöste Problem der Racemisierung, die insbesondere bei Segmentkondensation mittels chemischer Verknüpfungsmethoden in Erscheinung tritt. Da sich Stereoisomere kaum vollständig trennen lassen und die optische Reinheit der Syntheseprodukte für die biologische Aktivität eine notwendige Voraussetzung ist, besitzt die industrielle Synthese von Peptiden mittels organisch-chemischer Verfahren erhebliche Nachteile. Weiterhin müssen bei allen chemischen peptidsynthetischen Operationen die Drittfunktionen von Aminosäurebausteinen wegen der Gefahr der Nebenreaktionen reversibel maskiert werden. Zur Umgehung der geschilderten Schwierigkeiten bietet sich der Einsatz von Biokatalysatoren für die Katalyse des Peptidknüpfungsschrittes an (vgl. Übersichten: Jakubke, H.-D. (1987) in S. Udenfriend, J. Meienhofer (Eds.): The Peptides: Analysis, Synthesis, Biology, Vol. 9, Academic New York, pp. 103 - 165; Kullmann (1987) Enzymatic Peptide Synthesis, CRC, Boca Raton, USA; Jakubke, H.-D., Kuhl, P. u. Könnecke, A. (1985) Angew. Chem. 97, 79). Durch die Stereospezifität der als Biokatalysator eingesetzten Proteasen bleibt die chirale Integrität erhalten und der hohe Grad der Reaktionskontrolle ermöglicht einen weitgehenden Verzicht auf den Schutz von Drittfunktionen. Im Rahmen der enzymkatalysierten Peptidsynthese kommt der kinetisch kontrollierten Reaktionsführung eine Schlüsselrolle zu (Schellenberger, V., Jakubke, H.-D. (1991) Angew. Chem. 103, 1440). Die bei dieser Methode mit der Peptidproduktbildung einhergehende Hydrolyse des Acylenzymintermediats und weitere mögliche proteolytische Spaltungen stellen noch für viele Synthesereaktionen ein Problem dar, indem die Ausbeute an Peptidprodukt begrenzt bleibt und die Abtrennung von Nebenprodukten die Herstellung erschwert.

Aufgabe der Erfindung ist die biokatalytische Herstellung von Peptiden unter möglichst vollständigem Ausschluß unerwünschter proteolytischer Nebenreaktionen.

Diese Aufgabe wird gelöst mit einem Verfahren gemäß Anspruch 1.

Gemäß der vorliegenden Erfindung erfolgt die biokatalytische Herstellung eines Peptids nicht wie gewöhnlich mit einer Protease, sondern mit einem Zymogen (H. Tschesche et al. in Methods of Enzymatic Analysis, 3rd Ed., Vol. V, Weinheim, S. 76-77). Da Zymogene proteolytisch noch inaktive Vorstufen (Proenzyme) von Proteasen sind und daher eigentlich keine katalytische Effizienz haben sollten, sind die erfindungsgemäßen Ergebnisse sehr überraschend. Für hohe Ausbeuten ist es vorteilhaft, vergleichsweise höhere Konzentrationen des gewählten Zymogens einzusetzen. Die gesamte Verfahrensweise, d. h. Wahl der Substrate, des Puffers, des Mediums, der Temperatur, der Konzentrationen, der Reaktionszeit, etc., ist verhältnismäßig unkritisch und kann vom Fachmann für biokatalytische Peptidsynthesen einfach ermittelt werden.

Vorzugsweise werden für die erfindungsgemäßen biokatalytische Peptidsynthesen die bei Peptidsynthesen mit Proteasen üblichen Substrate eingesetzt. Geeignet sind insbesondere ggf. N-geschützte Aminosäureester bzw. ggf. N-geschützte Peptidester, die man mit einer C-terminal geschützten Aminosäure umsetzt. Die NH₂-Gruppe der Acyldonorester wird gewünschtenfalls durch in der Peptidchemie übliche Aminoschutzgruppen, wie Boc-, Z-, etc. oder durch Acylierung geschützt, geeignet sind z. B. Maleinsäure, Essigsäure, Benzoesäure, etc. Als Ester kommen insbesondere die niederen Ester in Frage, besonders geeignet sind die Methyl- und Ethylester, aber auch Cyanmethylester, p-Nitrobenzylester, Carboxamidomethylester,

Als C-terminale Komponente eignen sich vorteilhaft C-terminal derivatisierte Aminosäuren oder Peptidfragmente, insbesondere Aminosäureamide, wobei auch Peptide, die ja ebenfalls Amide sind, eingesetzt werden können. Beim Einsatz von Peptiden kann das C-terminale Ende gewünschtenfalls geschützt sein, z. B. durch eine NH₂-Gruppe.

Die Wahl eventueller Derivatisierungen und Ausgangssubstrate richtet sich dabei grundsätzlich nach dem gewünschten Endprodukt und dem eventuellen Erfordernis, Nebenreaktionen zu verhindern. Diese Wahl liegt daher im üblichen fachmännischen Handeln.

Das erfindungsgemaße Verfahren offenbart gegenüber der bisherigen Verwendung von Proteasen den besonderen Vorteil der Unterdrückung von unerwünschten proteolytischen Spaltreaktionen. Dies gelingt erfindungsgemäß, da das Zymogen gegenüber der entsprechenden Protease eine neue Spezifität zeigt. Hierdurch wird es möglich, daß man für einen gewünschten Peptidaufbau ein solches Zymogen wählt, das einerseits geeignet ist, aus den Substraten in annehmbarer Zeit und Ausbeute das Peptid aufzubauen, und das andererseits mit einem solchen Enzym korrespondiert, für das das erhaltene Peptid vorzugsweise kein Substrat ist, d. h., daß der Peptidaufbau (Verknüpfungsgeschwindigkeit) durch das Zymogen mindestens eine Größenordnung schneller sein soll als der Peptidabbau durch das dem Zymogen korrespondierende Enzym. Mit anderen Worten, vorzugsweise wird das Zymogen so gewählt, daß die Verknüpfungsgeschwindigkeit der gewählten Substrate vorzugsweise zehnmal höher ist als die Geschwindigkeit des Peptidabbaus durch das dem Zymogen korrespondierende Enzym. So ist es beispielsweise möglich, mit Trypsinogen "Chymotrypsin-spezifische" Substrate oder mit Chymotrypsinogen "Trypsinspezifische" Substrate umzusetzen. Hierdurch wird vermieden, daß das Zymogen zu einem geringen Prozentsatz begleitende korrespondierende Enzym oder sogar bei der Synthese durch limitierte Proteolyse aus dem Zymogen entstehende Enzym zu einer Rückreaktion führt. Diese Art der Sekundärhydrolyse kann durch diese Verfahrensweise weitgehend oder vollständig unterbunden werden.

Vorzugsweise werden als Zymogen Chymotrypsinogen, Trypsinogen, Pepsinogen, Prorennin oder Pro-Carboxypeptidase eingesetzt.

Erfindungsgemäß werden Peptide bevorzugt hergestellt aus einer freien oder α-Aminogruppen-geschützten Aminosäure oder einem freien oder α-Aminogruppen-geschützten Peptid, dessen in Reaktion tretende Carboxylgruppe als Ester vorliegt, und einer Aminosäure, einem Aminosäurederivat oder einem Peptid, bei dem die in Reaktion tretende Aminosäure am NH₂-Ende unblockiert ist, in Gegenwart des Zymogens, z. B. des Zymogens einer Serinprotease in Lösung oder Suspension bei Raumtemperatur, die ggf. Anteile organischer Lösungsmittel und/oder Puffersubstanzen enthält, oder auch tieferen Temperaturen.

Vorzugsweise wird die Reaktion in einem wässrigen Medium durchgeführt, das ggf. Anteile organischer Lösungsmittel enthalten kann, wobei u. U. tiefe Temperaturen vorteilhaft sind, bei denen auch das wässrige Medium zu Eis gefroren sein kann, da bei der Durchführung des erfindungsgemäßen Verfahrens bei tiefen Temperaturen zusätzlich die Sekundärhydrolyse unterdrückt werden kann.

Die gebildeten Peptide können mit geeigneten chromatographischen oder extraktiven Techniken präparativ separiert werden. Nach beendeter Aufarbeitung können ggf. noch vorhandene Schutzgruppen nach bekannten Methoden entfernt werden.

Als Puffer können auch solche eingesetzt werden, die proteolytische Reaktionen inhibieren. Auch hierdurch können unerwünschte Sekundärhydrolysen reduziert werden.

Im Gegensatz zu beschriebenen Verfahren zur Synthese von Peptiden unter Nutzung von Proteasen wird durch den erfindungsgemäßen Einsatz von proteolytisch inaktiven Proenzymen die Gefahr unerwünschter Spaltungen signifikant vermindert. Erstmalig wird für die Stoffklasse der Peptide ihre biokatalytische Synthese mit Zymogenen durchgeführt. Der Effekt der Erfindung ist insofern überraschend als mit proteolytisch inaktiven Proenzymen eine Peptidbindungsknüpfung nicht erwartet werden konnte. Bei Anwesenheit von geringen Anteilen aktiver Proteasespecies in den eingesetzten Zymogenen wird durch Temperaturerniedrigung bzw. Einfrieren der Reaktionslösung in bekannter Weise eine mögliche proteolytische Nebenreaktion praktisch ausgeschaltet: D. h., daß mit den oben beschriebenen
Verfahrensvarianten insbesondere die kommerziell zur Verfügung stehenden Zymogene, die meist wenige Gew.-Prozente des korrespondierenden Enzyms enthalten, eingesetzt werden können.

Die vorliegende Erfindung zeigt neue
Verwendungsmöglichkeiten für Zymogene, mit zur Erfindung gehört daher auch die Verwendung eines Zymogens zur Peptidsynthese.

Die mit der vorliegenden Erfindung herstellbaren Peptide oder -derivate eignen sich, ggf. nach Abspaltung von Schutzgruppen, z. B. als Wirkstoffe bzw. Zwischenprodukte von Wirkstoffen.

Die vorliegende Erfindung wird im folgenden anhand von Beispielen näher ausgeführt.

In den Beispielen werden die Aminosäuren nach international gültigen Regeln abgekürzt. Zusätzlich werden folgende Abkürzungen verwendet:

| | |
|---|---|
| Ac | Acetyl |
| Bz | Benzoyl |
| Boc | tert.-Butyloxycarbonyl |
| Mal | Maleyl |
| OCam | Carboxamidomethylester |
| OEt | Ethylester |
| OMe | Methylester |
| NH₂ | Amid |

Wenn nicht anders vermerkt, besitzen Aminosäuren bzw. Aminosäurereste mit chiralem Zentrum L-Konfiguration.

### Beispiel 1

### Synthese von Mal-Phe-Leu-NH₂

**a) mit Trypsinogen**
   2 ml 0,05 M Borat-Puffer, pH 9, enthaltend 0,2 M NaCl, 2 mM Mal-Phe-OMe, 50 mM H-Leu-NH₂ und 4 µM Trypsinogen werden bei 25°C unter pH-Kontrolle gerührt. Nach 24 h wird die Reaktionslösung mit 0,5 proz.
   Trifluoressigsäure in Methanol/Wasser (1 : 1; v/v) auf pH 2 gebracht. Die Ausbeute wird HPLC analytisch bestimmt und beträgt 73,5 % d. Th.
   Führt man die Synthese nicht bei Raumtemperatur, sondern bei -15°C im gefrorenen wäßrigen System durch, dann beträgt die Ausbeute nach 24 h bei einem C/N-Verhältnis von 1 : 10 87 % d. Th. bzw. bei einem C/N von 1 : 5 77 % d. Th.
**b) mit Chymotrypsinogen**
   Wie in Beispiel 1a, jedoch mit 4 µM Chymotrypsinogen als Biokatalysator. Ausbeute nach 24 h: 82 % d. Th. Ein Vergleichsansatz führte nach 48 h zu einer Ausbeute von 80,3 % d. Th.
   Führt man die Synthese unter pH-Stat-Bedingungen (KOH; pH 8,5) in Abwesenheit von Borat-Puffer und NaCl durch, dann beträgt die Ausbeute nach 2 h 83 % d. Th. Ein Vergleichsansatz fürhte nach 72 h zu unveränderter Ausbeute.

### Beispiel 2

### Synthese von Mal-Phe-Leu-Leu-NH₂

2 ml 0,05 M Borat-Puffer, pH 9, enthaltend 0,2 M NaCl, 2 mM Mal-Phe-OMe, 50 mM H-Leu-Leu-NH₂ und 12 µM Trypsinogen werden bei 25°C unter pH-Kontrolle gerührt. Die Reaktion wird wie unter 1a beschrieben abgestoppt. Nach 24 h beträgt die HPLC analytisch bestimmte Ausbeute 61 % d. Th. Ein Parallelansatz führte nach 48 h zu einer Ausbeute von 60,3 % d. Th.

### Beispiel 3

### Synthese von Mal-Phe-Phe-Leu-NH₂

1 ml 0,1 M Veronal-Na/HCl-Puffer, pH 8.3/56 % (v/v) DMSO, erhaltend 40 mM Mal-Phe-Phe-OCam, 120 mM H-Leu-NH₂ und 40 µM Trypsinogen werden bei Raumtemperatur unter pH-Kontrolle gerührt. Nach 85 min wird die Reaktionslösung mit 2 proz. Essigsäure abgestoppt. Die Ausbeute wird HPLC analytisch bestimmt und beträgt 72 % d. Th.

### Beispiel 4

### Synthese von Boc-Phe-Leu-NH₂

1 ml 0,1 M Veronal-Na/HCl-Puffer, pH 8,3/50 % (v/v) DMSO, enthaltend 100 mM Boc-Phe-OCam, 300 mM H-Leu-NH₂ und 100 µM Trypsiongen, werden bei Raumtemperatur unter pH-Kontrolle gerührt. Nach 3 h wird die Reaktion wie unter 3 beschrieben gestoppt. Die Ausbeute wird HPLC analytisch bestimmt und beträgt 93 % d. Th.

### Beispiel 5

### Synthese von Ac-Tyr-Leu-NH₂

**a) mit Chymotrypsinogen**
   0,5 ml 0,05 M Borat-Puffer, enthaltend 0,2 H NaCl, 2 mM Ac-Tyr-OEt, 50 mM H-Leu-NH₂ und 4 µM Chymotrypsinogen werden bei 25°C unter pH-Kontrolle gerührt. Die Reaktion wird wie unter 1a beschrieben gestoppt. Nach 30 min beträgt die HPLC analytisch bestimmte Ausbeute 83,5 %. Ein Parallelansatz führte nach 1 h zur gleichen Ausbeute, während nach 68 h Reaktionszeit nur 59 % d. Th. betrug.
   Führt man dagegen die Synthese unter gleichen Bedingungen, jedoch bei -15°C im gefrorenen Zustand durch, dann beträgt die Ausbeute nach 24 h 93 % d. Th.
**b) mit Trypsinogen**
   Wie im Beispiel 5a jedoch mit 12 µM Trypsinogen als Biokatalysator. Ausbeute nach 10 min: 76 % d. Th. Während Parallelansätze nach 30 min bzw. 60 min zu gleichen Ausbeuten führten, wurde nach 68 h nur 66 % d. Th. erhalten.
   Führt man dagegen die Synthese bei -15°C im gefrorenen Zustand durch, dann werden nach 1 h 45 % und nach 24 h sogar 93 % d. Th. erhalten.

### Beispiel 6

### Synthese von Bz-Arg-Leu-NH₂

**a) mit Trypsinogen**
   2 ml 0,05 M Borat-Puffer, pH 9, enthaltend 0,2 M NaCl, 2 mM Bz-Arg-OEt, 50 mM H-Leu-NH₂ und 4 µM Trypsinogen werden bei 25°C unter pH-Kontrolle gerührt. Die Reaktion wird wie beschrieben gestoppt. Nach 5 min beträgt die HPLC analytisch bestimmte Ausbeute 72 % d. Th. Parallelansätze führten nach 1 h bzw. nach 24 h zu 62 % bzw. 0 % Ausbeute.
   Führt man die Synthese nicht bei 25°C, sondern bei -15°C im gefrorenen Zustand durch, dann beträgt die Ausbeute bereits nach 1 h 93 % d. Th. Ein Parallelansatz führte nach 24 h unter den gleichen Bedingungen ebenfalls zu 93 % Ausbeute.
**b) mit Chymotrypsinogen**
   Wie im Beispiel 6a, jedoch mit 25 µM Chymotrypsinogen als Biokatalysator. Die HPLC analtytisch bestimmte Ausbeute beträgt nach 24 h bei Raumtemperatur 50 % d. Th., bzw. nach 72 h 66 % d. Th.

### Beispiel 7

### Synthese von Bz-Arg-Ala-Ile-OH

**a) mit Trypsinogen**
   2 ml 0,05 M Borat-Puffer, pH 9, enthaltend 0,2 M NaCl, 2 mM Mal-Phe-OMe, 50 mM H-Ala-Ile-OH und 4 µM Trypsinogen werden bei 25°C unter pH-Kontrolle gerührt. Die Reaktion wird wie beschrieben gestoppt. Nach 10 min beträgt die Ausbeute 68 % d. Th. Parallelansätze führten nach 1 h bzw. nach 24 h zu 58 bzw. 0 % Ausbeute.
   Führt man die Synthese nicht bei 25°C, sondern bei -15°C im gefrorenen Zustand durch, dann beträgt die Ausbeute nach 1 h 76 % d. Th. Ein Parallelansatz führte nach 24 h zu einer übereinstimmenden Ausbeute.
**b) mit Chymotrypsinogen**
   Wie im Beispiel 7a, jedoch mit 4 µM Chymotrypsinogen als Biokatalysator. Die Ausbeute beträgt nach 24 h 65 % bzw. nach 72 h 68 % d. Th.

### Beispiel 8

### Synthese von H-Phe-Arg-NH₂

**a) mit Chymotrypsinogen**
   1 ml 0,05 m Veronal-Natriumcarbonat-Puffer, pH 9, enthaltend 0,2 M NaCl, 2 mM Phe-OMe, 50 mM Arg-NH₂ und 10 µM Chymotrypsinogen werden bei Raumtemperatur gerührt. Nach 4 h wird die Reaktion mit 2,5 proz. Trifluoressigsäure gestoppt. Die Ausbeute wird HPLC analytisch bestimmt und beträgt 77 % d. Th. Ein Parallelansatz führte nach 24 h zu unveränderter Ausbeute.
**b) mit Trypsinogen**
   Wie im Beispiel 8a, jedoch mit 10 µM Trypsinogen. Die Ausbeute beträgt nach 4 h 61 % d. Th.

## Patentansprüche

1. Verfahren zur biokatalytischen Herstellung eines Peptids aus einem Aminosäureester oder einem Peptidester und einer C-terminal derivatisierten Aminosäure oder einem Peptidfragment,
**dadurch gekennzeichnet,**
daß man als Biokatalysator ein Zymogen einsetzt.

2. Verfahren nach Anspruch 1,
**dadurch gekennzeichnet,**
daß man das Peptid aus Substraten mittels eines Zymogens aufbaut, das so gewählt wird, daß die Geschwindigkeit des Peptidabbaus durch das dem Zymogen korrespondierende Enzym mindestens um eine Größenordnung langsamer ist als die Verknüpfungsgeschwindigkeit der gewählten Substrate.

3. Verfahren nach einem der vorhergehenden Ansprüche,
**dadurch gekennzeichnet,**
daß man die Reaktion in einem wässrigen Medium, das Anteile organischer Lösungsmittel enthalten kann, durchführt.

4. Verfahren nach Anspruch 3,
**dadurch gekennzeichnet**,
daß man die Reaktion in einem gefrorenen wässrigen Medium durchführt.

5. Verfahren nach einem der vorhergehenden Ansprüche,
**dadurch gekennzeichnet**,
daß man als Zymogen Chymotrypsinogen, Trypsinogen, Pepsinogen, Prorennin oder Pro-Carboxypeptidase einsetzt.

6. Verwendung eines Zymogens zur Peptidsynthese.

## Claims

1. Process for the biocatalytic production of a peptide from an amino acid ester or a peptide ester and a C-terminally derivatised amino acid or a peptide fragment,
characterised in that
a zymogen is used as the biocatalyst.

2. Process according to claim 1,
characterised in that
the peptide is synthesised from substrates by means of a zymogen, which is selected such that the rate of peptide degradation by the enzyme corresponding to the zymogen is at least one order of magnitude slower than the rate of linkage of the selected substrates.

3. Process according to one of the preceding claims,
characterised in that
the reaction is performed in an aqueous medium, which may contain fractions of organic solvent.

4. Process according to claim 3,
characterised in that
the reaction is performed in a frozen aqueous medium.

5. Process according to one of the preceding claims,
characterised in that
chymotrypsinogen, trypsinogen, pepsinogen, prorennin or procarboxypeptidase is used as the zymogen.

6. Use of a zymogen for peptide synthesis.

## Revendications

1. Procédé de fabrication biocatalytique d'un peptide à partir d'un ester d'aminoacide ou d'un ester de peptide, et d'un aminoacide converti en dérivé sur l'extrémité carbonée ou d'un fragment de peptide,
caractérisé en ce qu'
on met en oeuvre comme biocatalyseur, un zymogène.

2. Procédé selon la revendication 1,
caractérisé en ce qu'
on construit le peptide à partir de substrats à l'aide d'un zymogène qui est choisi de telle sorte que la vitesse de dégradation du peptide par l'enzyme correspondant au zymogène, est plus lente d'au moins un ordre de grandeur que la vitesse de couplage des substrats choisis.

3. Procédé selon l'une des revendications précédentes,
caractérisé en ce qu'
on effectue la réaction en milieu aqueux qui peut contenir des quantités de solvant organique.

4. Procédé selon la revendication 3,
caractérisé en ce qu'
on effectue la réaction dans un milieu aqueux congelé.

5. Procédé selon l'une des revendications précédentes,
caractérisé en ce qu'
on met en oeuvre comme zymogènes, du chymotrypsinogène, du trypsinogène, du pepsinogène, de la prorennine ou une procarboxypeptidase.

6. Utilisation d'un zymogène en vue de la synthèse peptidique.
